# EUROPEAN PATENT SPECIFICATION

(11) **EP 0 844 004 B1**
(45) Date of publication and mention of the grant of the patent: **12.09.2007**
(21) Application number: 96921138.2
(22) Date of filing: 02.07.1996
(51) Int. Cl.: A61K 48/00, A61K 47/42

(54) **COLLAGEN CONTAINING GENE PREPARATIONS**
ATELOCOLLAGEN ENTHALTENDE GEN-ZUSAMMENSETZUNGEN
PREPARATIONS DE GENES CONTENANT D' ATELOCOLLAGENE

(30) Priority: 03.07.1995 JP 16774495
(43) Date of publication of application: 27.05.1998
(73) Proprietor: KOKEN CO., LTD., Toshima-ku, Tokyo (JP); Dainippon Sumitomo Pharma Co., Ltd., Osaka-fu (JP)
(72) Inventor: TERADA, Masaaki, Tokyo 177 (JP); OCHIYA, Takahiro National Cancer Center Research Institute Tsukiji, Tokyo 104 (JP); MIYATA, Teruo, Tokyo 161 (JP); ITOH, Hiroshi, Tokyo 171 (JP)
(74) Representative: Vossius & Partner
(86) International application number: PCT/JP1996/001824
(87) International publication number: WO 1997/002047

(56) References cited:
- WO-A-92/20400
- WO-A-94/23699
- WO-A-95/24929
- JP-A- 1 068 278
- JP-A- 3 093 716
- JP-A- 3 163 032
- JP-A- 60 126 217
- JP-A- 61 236 729
- JP-A- 62 228 028

## Description

### FIELD OF THE INVENTION

The present invention relates to the field of medicine, especially gene therapy. Especially, the present invention relates to a preparation containing a gene. The preparation retains a gene vector or a gene stably, releases it gradually and maintains the therapeutic effect for a long time when administered into a living body. The preparation makes it possible to stop a treatment at favorable period.

### BACKGROUND OF THE INVENTION

Gene therapy is an active area of research, and expectation for successful therapy is extremely high. In gene therapy approaches in which the gene per se is the medicine, the therapy for disease is conducted by transferring a gene of a designated enzyme, cytokine, and the like into cells of a patient. The introduced gene then produces the designated substance in the patient's body. This gene therapy can be divided to two types. The purpose of the first type is semipermanent expression of designated gene by integrating the gene into genome of a patient. The purpose of another type is transient expression of a gene without expectation of its integration into genome. In the latter type of gene therapy, a method using adenovirus and the like is often adopted as a method for transferring into the patient a gene encoding, for example, a cytokine which increases immuno-potency against cancer cells (Cardiovascular Research, 28, 445 (1994); Science, 256, 808 (1992) ; Gastroenterology, 106, 1076 (1994); TIBTECH, 11, 182 (1993) ; J. Biol. Chem., 266, 3361 (1991) ; Nature Medicine, 1, 583, (1995); and references cited therein).

In the case of using a virus vector, though the efficiency of gene transfer is generally high, repeated administrations are difficult due to immune responses (J. Biol. Chem., 269, 13695 (1994), Am. J. Respir. Cell Mol., 10, 369 (1994)).

A preparation using collagen for gradually releasing medicines containing organic compounds or a protein preparation is disclosed in J. Controlled Release 33, 307-315 (1995), etc. However, the disclosed and usual medicine (for example, protein drug, medicine chemically synthesized, etc.) is dissolved in approximately 1-2 days when retained in, for example, collagen gel.

WO 94/23699 discloses different carrier-molecules promoting the uptake or transport of oligo deoxyribonucleotides into the target cell in order to control gene expression in animals or humans. However, the group of suggested carriers does not comprise soluble collagens (e.g. atelocollagen).

In a method which is presently used in gene therapy and comprises administering a gene vector (a gene-inserted vector) or a gene directly, the gene vector or the gene contacts cells immediately after administration, and immediately after that gene transfer starts and is completed at once. Furthermore, the gene transduced into cells is diluted (that is, its copy number decreases) with cell division or is reduced by degradation in cells. Therefore, expression of the transferred gene can be maintained only for several weeks which is too short to practice sufficient treatment and this is a deficiency in the method. Accordingly, repeated administrations of a gene vector or a gene are necessary. Therefore, an object of the present invention is to overcome these defects and provide a preparation which can release a gene vector or a gene gradually and can maintain the therapeutic effect for a long time.

Furthermore, a method enabling repeated administrations is expected because those are difficult in gene therapy using a virus vector and the like. Accordingly, a second object of the present invention is to provide a gene preparation enabling repeated administrations in gene therapy using a virus vector and the like.

Furthermore, it is desirable that gene expression in the body can be stopped any time to ensure safety, because a gene is expressed for several weeks which is longer than the term for a protein preparation. Accordingly, a third object of the present invention is to provide a gene preparation which can make it possible to quickly stop the gene transfer when termination of treatment is intended.

### SUMMARY OF THE INVENTION

The present inventors have examined various preparations for gradually releasing a gene vector or a gene and have found that, when a preparation wherein a gene or a vector inserted with a gene is incorporated into a carrier made of a biocompatible material was administered into a living body, the gene was unexpectedly expressed for many months. The present inventors have also found that the preparation can be administered repeatedly into a living body and thus the present invention has been accomplished.
Therefore, the characteristic features of the present invention are as follows.
(1) A sustained release gene preparation in a gel form comprising an atelocollagen and an intended gene or a vector comprising said intended gene.
(2) The sustained gene release preparation of claim 1, wherein said atelocollagen is present in the amount of 0.01-25 w/w% of the preparation.
(3) The sustained gene release preparation of claim 1, wherein said atelocollagen is present in the amount of 0.05-10 w/w% of the preparation.
(4) The sustained gene release preparation of any one of claims 1 to 3, wherein said preparation further comprises an additive.
(5) The sustained gene release preparation of claim 4, wherein said atelocollagen is present in the amount of 2 w/w% or more of the preparation, and said additive is present in the amount of 5-900 w/w% of the atelocollagen.
(6) A sustained release gene preparation in a film form obtainable by drying a gel comprising 0.2-30 w/w% of an atelocollagen, and an intended gene or a vector comprising said intended gene.
(7) The sustained gene release preparation of claim 6, wherein the atelocollagen is 0.3-5 w/w% in the gel.
(8) The sustained gene release preparation of claim 6, wherein the atelocollagen is 0.4-2 w/w% in the gel.
(9) The sustained gene release preparation of any one of claims 6 to 8, wherein said gel further comprises an additive.
(10) The sustained gene release preparation of claim 9,
   wherein said atelocollagen is 1% or more in the gel, and the additive is 5-900 w/w% of said atelocollagen.
(11) A sustained release gene preparation in a solid rod form obtainable by drying a gel comprising 10-30 w/w% of an atelocollagen, and an intended gene or a vector comprising said intended gene.
(12) The sustained gene release preparation of claim 11, wherein said gel further comprises an additive.
(13) The sustained gene release preparation of claim 12, wherein said additive is present in the amount of 5-100 w/w% of the atelocollagen.
(14) A pharmaceutical composition comprising the gene preparation of any one of claims 1 to 13, and optionally a pharmaceutically acceptable carrier.
(15) A sustained release gene preparation in a rod form obtainable by the process comprising:
   obtaining a sponge by lyophilizing a gel comprising 0.05 to 5 w/w% of atelocollagen, and an intended gene or a vector comprising said intended gene;
   obtaining a gel in which the concentration of atelocollagen is 10 to 30 w/w% by adding distilled water to the sponge;
   forming a rod by squeeze molding the gel; and
   drying.
(16) Use of the gene preparation of any one of claims 1 to 13 or obtainable by the process of claim 15 for the preparation of a pharmaceutical composition for delivering a gene for uptake by a cell.
(17) The use of claim 16, wherein said pharmaceutical composition is to be administered subcutaneously, intramuscularly or intraperitoneally to a subject.

### BRIEF DESCRIPTION OF THE DRAWINGS

Figure 1 is a graph showing a time course of the number of platelets in Test Example 1 and Comparative Example 1.
Figure 2 is a graph showing a time course of HST-1 in peripheral blood in Test Example 1 and Comparative Example 1.
Figure 3 is a graph showing a time course of the number of platelets in Test Example 4 and Comparative Example 1.
Figure 4 is a graph showing a time course of the number of platelets in Test Example 5.
Figure 5 is a graph showing dose-dependency of collagen on the amount of HST-1 in peripheral blood in Test Example 6.
Figure 6 is a graph showing a time course of the number of platelets in Test Example 7 and Comparative Example 2.

### DETAILED DESCRIPTION OF THE INVENTION

The present invention will be described below in more detail.

"A vector for gene insertion" may be any vector that can transfer a gene into cells and includes, for example, a virus vector, a liposome vector, a fusogenic liposome vector in which a virus and a liposome are fused, or the like (Cardiovascular Research, 28, 445 (1994); Science, 256, 808 (1992); Gastroenterology, 106, 1076 (1994); TIBTECH, 11, 182 (1993); J. Biol. Chem., 266, 3361 (1991); Nature Medicine, 1, 583, (1995); and references cited therein).

A virus vector may be any vector that can be used as an ordinary vector in gene therapy and includes, for example, an adenovirus, an adeno-associated virus, a vaccinia virus, a retrovirus, an HIV virus, a herpesvirus, or the like. The gene vector can be obtained by inserting a gene for transfer into a virus vector directly according to a conventional method, for example, described in the above references.

A liposome vector may be any vector that can be used as an ordinary liposome vector in gene therapy and includes, for example, a liposome vector obtained by mixing DOTMA, DOPE, DOGS, etc. When a cationic liposome vector is used, transfer efficiency into cells is high. Examples of the fusogenic liposome vector in which a virus and a liposome are fused include a fusogenic liposome vector in which a Sendai virus (HVJ: hemagglutinating virus of Japan) and a liposome are fused, and the like. A gene vector can be obtained by enclosing a gene for transfer into a liposome vector or a fusogenic liposome vector according to a conventional method, for example, described in the above references. The enclosed gene may be in any form that can express the gene in living body and is preferably a form stable in a living body such as a plasmid, etc.

A gene itself can be retained in a gene preparation of the present invention without being inserted into "a vector for gene insertion" which transfers a gene into cells. In this case, a form of a gene may be any form that can express the gene in living body. For example, a gene may be inserted into what is called an expression vector and the like. A form of a gene is preferably a form stable in a living body such as a plasmid, etc.

"A gene" for transfer may be any gene that can be used in gene therapy and includes, for example, an adenosine deaminase gene, a GM-CSF gene, a thymidine kinase gene, or the like.

"Biocompatible material" is the material which has high biocompatibility and can retain a gene vector or a gene stably in living body.

The biocompatible material to be used is atelocollagen.

"An additive" may be added to a preparation of the present invention according to necessity in order to stabilize a gene vector and the like, accelerate gene transfer into cells or nuclei or regulate the release of a gene vector and the like. An additive may be any additive that can achieve the purpose and includes, for example, sucrose, glycine, chondroitin sulfate, gelatin, albumin, cytokine, a mixture of the High Mobility Group Proteins HGM-1 and HCM-2 (High Mobility Group-1,-2 Mixture; Experimental Medicine, 12, 184 (1994), BIOTHERAPY, 8, 1265 (1994)), chloroquine, polylysine (Hepatology, 22, 847 (1995)), Transfectam (Trademark, Wako Pure Chemical Industries, Ltd.), or the like.

In the case wherein atelocollagen is mixed with another biocompatible material or an additive, the content of collagen may be at least 10 w/w%, preferably at least 30 w/w% more preferably at least 50 w/w%, and most preferably at least 70 w/w%.

The content of biocompatible material in the gene preparation is varied depending on size, kind and the like of the gene vector or the gene and kind and the like of the biocompatible material.

A preferred content of biocompatible material in a gene preparation is 0.01-30 w/w% under the condition that the gene preparation is in a living body, more preferably 0.05-10 w/w%, and most preferably 0.05-5 w/w%.

Furthermore, the content of biocompatible material in the gene preparation is varied depending on the particular preparation.

When the preparation is in gel form, a preferred content of atelocollagen is 0.01-25 w/w%, more preferably 0.05-10 w/w%, most preferably 0.05-5 w/w%. However, when the content of atelocollagen is 2 % or more, it is preferred that an additive is added at 5-900 w/w% of atelocolllagen.

When the preparation is in film form, a preferred content of atelocollagen is 0.2-30 w/w% as a content before drying, more preferably 0.3-5 w/w%, most preferably 0.4-2 w/w%. However, when a content of atelocollagen is 1 % or more, it is preferred that an additive is added at 5-900 w/w% of atelocollagen.

When the preparation is in a solid rod form, the preferred content of atelocollagen is 10-30 w/w% and it is preferred that an additive is added at 5-100 w/w% of collagen.

A gene preparation of the present invention is not limited to a specific form. The preparation can be in solution, suspension, water-containing gel, film or sponge. Solid forms can be shaped as a rod, sphere and the like. However, a preferred form is generally a solution, suspension or water-containing gel, though the form of the preparation is varied depending on kind, size and the like of a vector for gene insertion.

A gene preparation is obtained by keeping a content of atelocollagen in the gene preparation within the above preferred range under the condition that the gene preparation is in a living body.

Examples of methods for producing a gene preparation in a form of solution, suspension or water-containing gel include (1) a method which comprises mixing a powder, solution, suspension or gel of a gene vector or a gene (referred to as a gene vector and the like below) with a carrier in a form of a solution or gel to which an additive is added according to necessity, (2) a method which comprises making a solution, suspension or gel of a gene vector and the like penetrate into a carrier that is in powder form to which an additive is added according to necessity, or (3) a method which comprises making a solution, suspension or gel of a gene vector and the like penetrate into a carrier that is a sponge to which an additive is added according to necessity and kneading them together. The methods for making a gene preparation of the present invention are not limited to such methods.

Examples of methods for producing a gene preparation in a solid form include (1) a method which comprises mixing a powder, solution, suspension or gel of a gene vector and the like with a carrier in a form of solution or gel to which an additive is added according to necessity and drying the mixture, (2) a method which comprises mixing a powder, solution, suspension or gel of a gene vector and the like with a carrier in powder form to which an additive is added according to necessity and drying the mixture, (3) a method which comprises making a solution, suspension or gel of a gene vector and the like penetrate into a carrier in the form of a sponge to which an additive is added according to necessity and drying the sponge, (4) a method which comprises making a solution, suspension or gel of a gene vector and the like penetrate into a carrier in the form of a sponge to which an additive is added according to necessity and drying the sponge as it stands or kneading and drying the sponge after adding water and the like according to necessity, (5) a method which comprises crushing and press-molding a solid product obtained by a method according to (1) to (4), or (6) a method which comprises mixing a powder of a gene vector and the like with a carrier that is a powder to which an additive is added according to necessity and press-molding the mixture. The present invention is not limited to such methods. The drying method; temperature and moisture at drying; mixing method; temperature and moisture at mixing; method for press-molding; temperature, moisture and compressing pressure at press-molding; solution velocity of carrier solution and gene vector solution; and solution velocity of mixture of carrier and gene vector solution; and pH may be as same as those in conventional methods.

A gene preparation of the present invention can be administered by various methods according to the disease being treated, the targeted organ and the like. For example, a gene preparation of the present invention can be administered subcutaneouly or intramuscularly, or can be administered directly to targeted sites of disease such as kidney, liver, lung, brain and the like. Direct administration to disease site enables organ-selective therapy.

Furthermore, repeated administrations are possible.

On the other hand, when discontinuance of gene transfer is required depending on the kind or condition of a disease, a gene preparation can be taken out as it stands, and gene transfer can be stopped. For example, if a gene preparation is a solid, it can be taken away by surgery or the like. When gene therapy is conducted by using a gene preparation wherein a gene vector and the like is retained in a vessel or the like having pores through which a virus can pass freely, the vessel or the like can be taken away at termination of the treatment. For example, gene therapy can be conducted by using a vessel (tube) as described in Japanese patent application No. 3(1991)-120115 (International publication number WO92/20400).

A gene preparation of the present invention can gradually release a gene vector and the like and simultaneously can retain a gene-inserted vector and the like stably in a living body during the sustained release. Therefore, the time of effective gene expression after a single administration can be extended by controlling the period of gene transfer into cells by delaying contact between cells and vectors.

A gene preparation of the present invention also enables repeated administrations of a gene vector such as a virus vector and the like which is otherwise difficult to administer repeatedly due to appearance of neutralizing antibodies in a subject.

Furthermore, a gene preparation of the present invention enables regulation of gene expression because the gene preparation can be taken away when termination of treatment is intended.

### Examples

### Example 1

A gene preparation was prepared by mixing 0.9 ml of culture medium containing 10⁹ pfu (plaque forming unit) of an adenovirus (Adex1 HST-1; Proc. Natl. Acad. Sci. USA, Vol. 91, 12368 (1994)) inserted with a gene encoding fibroblast cell growth factor HST-1 (FGF4) (prepared according to the method described in Proc. Natl. Acad. Sci. USA, 84, 2980-2984 (1987)) with 0.1 ml of neutral solution of atelocollagen (atelocollagen implant produced by KOKEN: 2% atelocollagen solution). The above adenovirus (Adex1 HST-1) can be obtained by deleting part of E1A, E1B and E3 of adenovirus type 5 (ATCC catalog No. VR-5) according to a method described in J. Virol., 54, 711-719 (1985) or Proc. Natl. Acad. Sci. USA, 93, 1320 (1996) and Cell, 13, 181-188 (1978); inserting a gene of fibroblast cell growth factor HST-1 into the non-proliferative-type adenovirus gene.

### Example 2

A gene preparation in gel form is prepared by mixing 0.1 ml of 2% atelocollagen neutral solution with 0.9 ml of culture medium containing 10⁹ pfu of Adex1 HST-1 and then keeping the mixture at 37 °C.

### Example 3

A gene preparation is prepared by obtaining a sponge by lyophilizing atelocollagen neutral solution; cutting the sponge about 5 mm of square; adding the cut sponge to 1 ml of culture medium containing 10⁹ pfu of Adex1 HST-1; and letting the sponge stand overnight.

### Example 4

A gene preparation is prepared by lyophilizing a gene preparation in gel form that is prepared in Example 2.

### Example 5

A gene preparation in pellet form (a compressed product in the shape of a rod) is prepared by again lyophilizing the gene preparation that is obtained in Example 3 and compressing the lyophilized sponge into a rod shape.

### Example 6

A gene preparation is prepared by mixing a plasmid vector which is obtained by inserting fibroblast cell growth factor HST-1 (FGF4) into an expression vector (pRc/CMV) having a cytomegalovirus (CMV) promoter with a liposome (DMRIE-C (produced by GIBCO-BRL)) and then mixing a solution containing the liposome with the same volume of atelocollagen neutral solution.

### Example 7

A gene preparation is prepared by enclosing the gene preparation which was obtained in Example 1 in a bag made of polyester (made from artificial blood vessel) (Micron (trademark, produced by INTERVASCULAR).

### Example 8

As same as Example 1, 1 ml of a gene preparation was prepared by mixing culture medium containing 10⁹ pfu of Adex1 HST-1 with atelocollagen neutral solution so as to come to be 0.1, 0.2, 0.4, 1.0 or 2.0 w/w% in final concentration of atelocollagen.

### Example 9

A gene preparation in gel form is prepared by mixing 5 ml of aqueous solution which contains a plasmid pOG44 (purchased from Stratagene) at a concentration of 73.25 µg/ml with 5 a of atelocollagen acid solution (containing 2 % atelocollagen, pH 3.5); lyophilizing the mixture to obtain a sponge; adding distilled water to the sponge so as to come be 0.4, 2, 5, 10 or 20 w/w% in a concentration of atelocollagen.

### Example 10

A gene preparation in gel form is prepared by mixing 5 ml of aqueous solution which contains a plasmid pOG44 at a concentration of 73.25 µg/ml with 5 g or 2.5 g of atelocollagen acid solution; adding 260 µl or 640 µl of aqueous solution of human serum albumin (80 mg/ml) to the mixture and mixing them; lyophilizing the mixture to obtain a sponge; adding distilled water to the sponge so as to come be 10 w/w% in the total concentration of atelocollagen and human serum albumin.

### Example 11

A gene preparation in a form of film is prepared by spreading and gradually drying the gene preparation in gel form that is obtained in Example 9 on a glass.

### Example 12

A gene preparation in rod form is prepared by squeeze-molding and gradually drying the gene preparation in gel form that is obtained in Example 9 or 10.

### Comparative Example 1

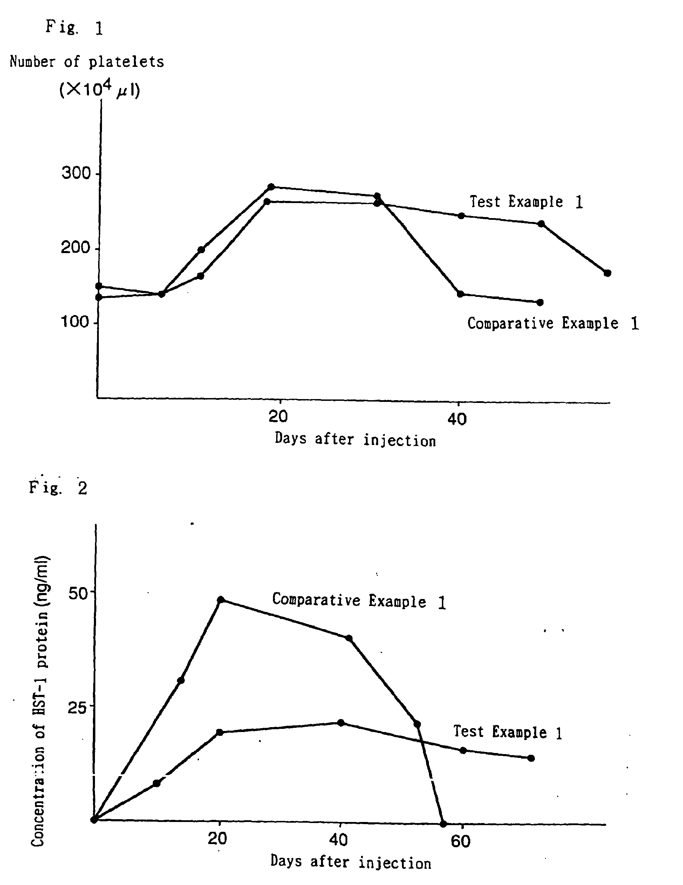

One ml of culture medium containing 10⁹ pfu of Adex HST-1 was administered intraperitoneally to a mouse. Then, on about the 12th day after administration, the number of platelets increased approximately two-fold, and this effect lasted to the 20-30th day. Furthermore, the concentration of HST-1 in peripheral blood was 50 ng/ml and at its maximum on the 20th day after administration. However, the concentration of HST-1 could not be maintained at a fixed level differently from the preparation of Example 1 below and HST-1 could not be detected in blood on the 60th day after administration. These results are shown in Figures 1 and 2.

### Test Example 1

1.0 ml of a gene preparation which had been prepared in Example 1 was administered intraperitoneally to a mouse. Then, on about the 12th day after administration, the number of platelets increased approximately two-fold, and this effect lasted beyond the 50th day. Furthermore, the concentration of HST-1 in peripheral blood was maintained at 20 ng/ml beyond the 80th day after administration. These results are shown in Figures 1 and 2.

### Test Example 2

1.0 ml of a gene preparation prepared as in Example 2 is administered intraperitoneally to a mouse. Then, on about the 12th day after administration, the number of platelets increases approximately two-fold, and this effect lasts beyond the 60th day after adminiatration.

### Test Example 3

A gene preparation prepared as in Example 3 is administered intraperitoneally to a mouse. Then, on about the 12th day after administration, the number of platelets increases approximately two-fold, and this effect lasts beyond the 60th day after administration.

### Test Example 4

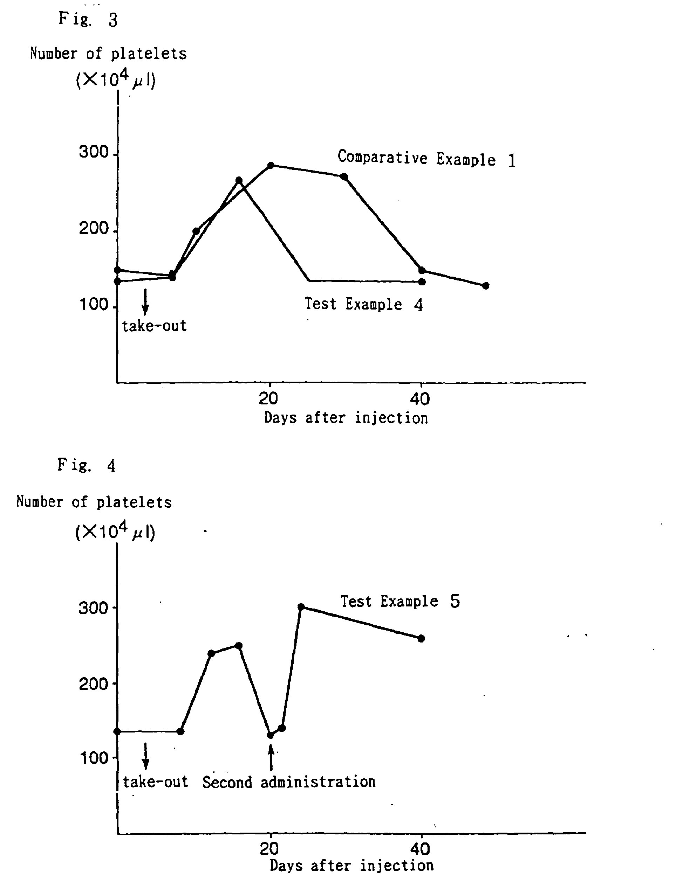

A gene preparation which had been prepared in Example 1 was administered intraperitoneally to a mouse, and the gene preparation was taken out on the third day after administration. As a result, on about the 12th day after administration the number of platelets approximately doubled, then decreased and returned to the normal level on about the 25th day after administration. These results are shown in Figure 3.

### Test Example 5

A gene preparation which had been prepared in Example 1 was administered intraperitoneally to a mouse and the gene preparation was taken out on the third day after administration. On the 20th day after administration a gene preparation which had been prepared in Example 1 was administered again. As a result, on about the 12th day after administration the number of platelets approximately doubled, then decreased and returned to the normal level on about the 20th day after administration. Immediately after the second administration, the number of platelets again increased approximately two-fold, and this effect lasted beyond the 40th day after the first administration. These results are shown in Figure 4.

### Test Example 6

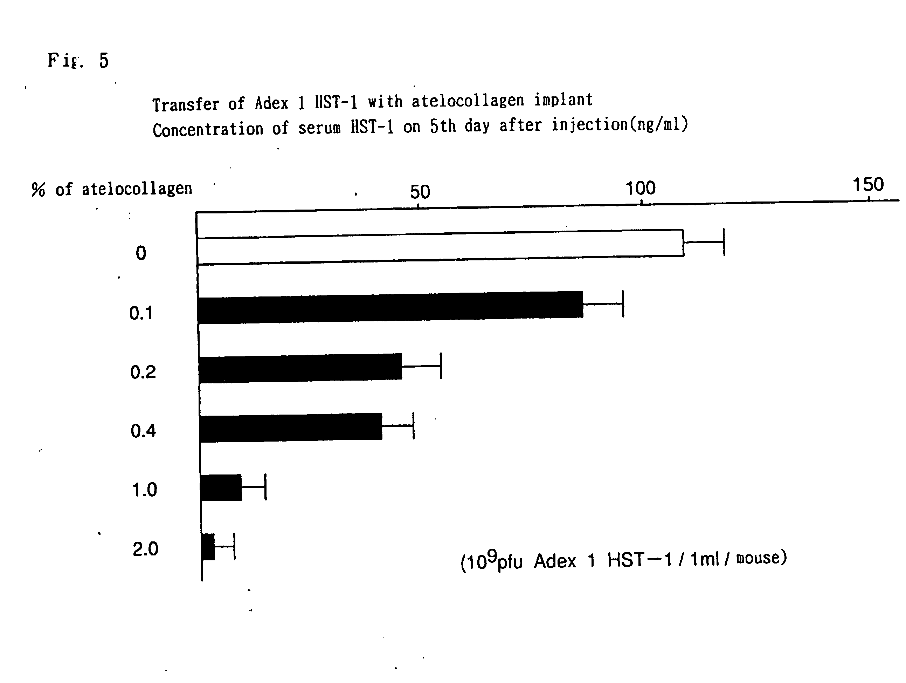

Each of 5 gene preparations which had been prepared in Example 10 or 1 ml of culture medium which contained 10⁹ pfu of Adex HST-1 and was completely free from collagen was administered intraperitoneally to a mouse. The concentration of HST-1 protein in peripheral blood was determined on the 5th day after administration. The results are shown in Figure 5. The data show that the serum concentration of HST-1 protein deceased with increasing concentration of collagen in the preparation.

### Test Example 7

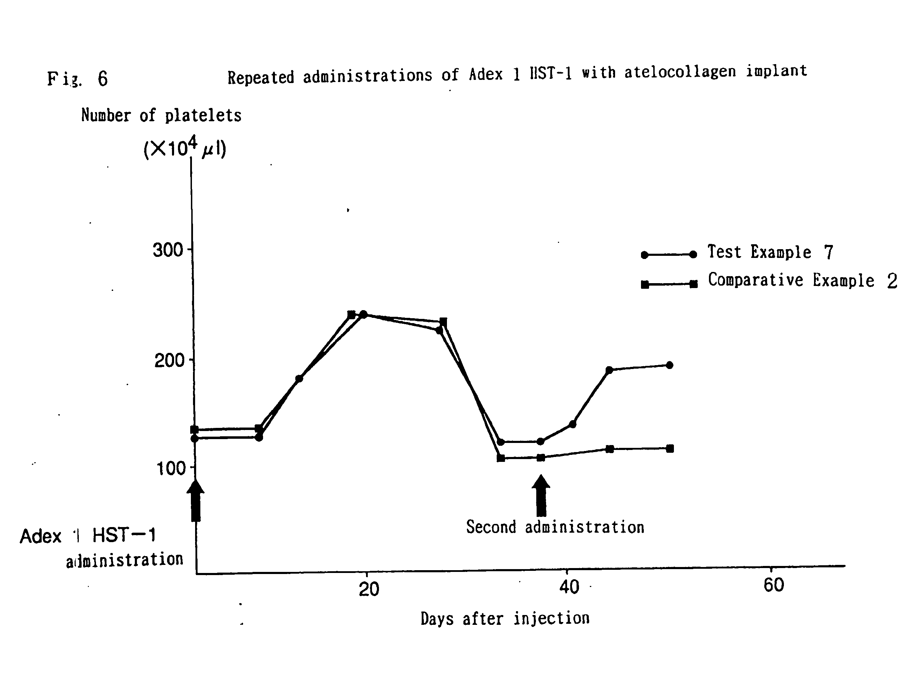

As a result that 1 ml of culture medium containing 10⁹ pfu of Adex HST-1 was administered intraperitoneally to a mouse, the number of platelets approximately doubled by the 12th day after administration, then decreased and returned to the normal level on about the 35th day after administration. On the 37th day after the first administration, 1 ml of an atelocollagen-containing gene preparation which had been prepared in Example 1 was administered again. Immediately after that, the number of platelets increased approximately two-fold, and this effect lasted throughout the remaining term of the experiment. These results are shown in Figure 6.

### Comparative Example 2

One ml of culture medium containing 10⁹ pfu of Adex HST-1 was administered intraperitoneally to a mouse and again administered on the 37th day after the first administration. As a result, on about the 12th day after the first administration the number of platelets approximately doubled, then decreased and returned to the normal level on about the 35th day after the first administration. Platelets did not increase after the second administration of Adex HST-1. These results are shown in Figure 6.

The results in Test Example 7 and Comparative Example 2 reveal that it is possible to administer a gene preparation prepared in Example 1 repeatedly in contrast with the result obtained upon repeated administrations of an adenovirus vector not formulated according to the present invention. The inability to make a repeated administration is due to the appearance of neutralizing antibodies.

## Claims

1. A sustained release gene preparation in a gel form comprising an atelocollagen and an intended gene or a vector comprising said intended gene.

2. The sustained gene release preparation of claim 1, wherein said atelocollagen is present in the amount of 0.01-25 w/w% of the preparation.

3. The sustained gene release preparation of claim 1, wherein said atelocollagen is present in the amount of 0.05-10 w/w% of the preparation.

4. The sustained gene release preparation of any one of claims 1 to 3, wherein said preparation further comprises an additive.

5. The sustained gene release preparation of claim 4, wherein said atelocollagen is present in the amount of 2 w/w% or more of the preparation, and said additive is present in the amount of 5-900 w/w% of the atelocollagen.

6. A sustained release gene preparation in a film form obtainable by drying a gel comprising 0.2-30 w/w% of an atelocollagen, and an intended gene or a vector comprising said intended gene.

7. The sustained gene release preparation of claim 6, wherein the atelocollagen is 0.3-5 w/w% in the gel.

8. The sustained gene release preparation of claim 6, wherein the atelocollagen is 0.4-2 w/w% in the gel.

9. The sustained gene release preparation of any one of claims 6 to 8, wherein said gel further comprises an additive.

10. The sustained gene release preparation of claim 9, wherein said atelocollagen is 1 % or more in the gel, and the additive is 5-900 w/w% of said atelocollagen.

11. A sustained release gene preparation in a solid rod form obtainable by drying a gel comprising 10-30 w/w% of an atelocollagen, and an intended gene or a vector comprising said intended gene.

12. The sustained gene release preparation of claim 11, wherein said gel further comprises an additive.

13. The sustained gene release preparation of claim 12, wherein said additive is present in the amount of 5-100 w/w% of the atelocollagen.

14. A pharmaceutical composition comprising the gene preparation of any one of claims 1 to 13, and optionally a pharmaceutically acceptable carrier.

15. A sustained release gene preparation in a rod form obtainable by the process comprising:
obtaining a sponge by lyophilizing a gel comprising 0.05 to 5 w/w% of atelocollagen, and an intended gene or a vector comprising said intended gene;
obtaining a gel in which the concentration of atelocollagen is 10 to 30 w/w% by adding distilled water to the sponge;
forming a rod by squeeze molding the gel; and
drying.

16. Use of the gene preparation of any one of claims 1 to 13 or obtainable by the process of claim 15 for the preparation of a pharmaceutical composition for delivering a gene for uptake by a cell.

17. The use of claim 16, wherein said pharmaceutical composition is to be administered subcutaneously, intramuscularly or intraperitoneally to a subject.

## Patentansprüche

1. Genpräparat mit verlängerter Freisetzung in Gelform, umfassend ein Atelokollagen und ein vorgesehenes Gen oder einen Vektor, der das vorgesehene Gen umfasst.

2. Genpräparat mit verlängerter Freisetzung nach Anspruch 1, wobei das Atelokollagen in einer Menge von 0,01 - 25 Gew./Gew.% des Präparats vorliegt.

3. Genpräparat mit verlängerter Freisetzung nach Anspruch 1, wobei das Atelokollagen in einer Menge von 0,05 - 10 Gew./Gew.% des Präparats vorliegt.

4. Genpräparat mit verlängerter Freisetzung nach einem der Ansprüche 1 bis 3, wobei das Präparat ferner einen Zusatz umfasst.

5. Genpräparat mit verlängerter Freisetzung nach Anspruch 4, wobei das Atelokollagen in einer Menge von 2 Gew./Gew.% oder mehr des Präparats vorliegt, und der Zusatz in einer Menge von 5 - 900 Gew./Gew.% des Atelokollagens vorliegt.

6. Genpräparat mit verlängerter Freisetzung in Form einer Folie, die erhältlich ist durch das Trocknen eines Gels, umfassend 0,2 - 30 Gew./Gew.% eines Atelokollagens und ein vorgesehenes Gen oder einen Vektor, der das vorgesehene Gen umfasst.

7. Genpräparat mit verlängerter Freisetzung nach Anspruch 6, wobei das Atelokollagen mit 0,3 - 5 Gew./Gew.% in dem Gel vorliegt.

8. Genpräparat mit verlängerter Freisetzung nach Anspruch 6, wobei das Atelokollagen mit 0,4 - 2 Gew./Gew.% in dem Gel vorliegt.

9. Genpräparat mit verlängerter Freisetzung nach einem der Ansprüche 6 bis 8, wobei das Gel ferner einen Zusatz umfasst.

10. Genpräparat mit verlängerter Freisetzung nach Anspruch 9, wobei das Atelokollagen mit 1 % oder mehr in dem Gel vorliegt und der Zusatz mit 5 - 900 Gew./Gew.% des Atelokollagens vorliegt.

11. Genpräparat mit verlängerter Freisetzung in Form eines festen Stabes, erhältlich durch das Trocknen eines Gels, umfassend 10-30 Gew./Gew.% eines Atelokollagens und ein vorgesehenes Gen oder einen Vektor, der das vorgesehene Gen umfasst.

12. Genpräparat mit verlängerter Freisetzung nach Anspruch 11, wobei das Gel ferner einen Zusatz umfasst.

13. Genpräparat mit verlängerter Freisetzung nach Anspruch 12, wobei der Zusatz in der Menge von 5-100 Gew./Gew.% des Atelokollagens vorliegt.

14. Arzneimittel, das das Genpräparat nach einem der Ansprüche 1 bis 13 und gegebenenfalls einen pharmazeutisch verträglichen Träger umfasst.

15. Genpräparat mit verlängerter Freisetzung in Stabform, erhältlich durch das Verfahren, welches umfasst:
Erhalten eines Schwammes durch Gefriertrocknung eines Gels, umfassend 0,05 bis 5 Gew./Gew.% eines Atelokollagens und ein vorgesehenes Gen oder einen Vektor, der das vorgesehene Gen umfasst;
Erhalten eines Gels, in welchem die Konzentration von Atelokollagen 10 bis 30 Gew./Gew.% beträgt, durch Hinzufügen von destilliertem Wasser zu dem Schwamm;
Formen eines Stabes durch Pressformen des Gels; und
Trocknen.

16. Verwendung des Genpräparats nach einem der Ansprüche 1 bis 13 oder erhältlich durch das Verfahren nach Anspruch 15 für die Herstellung eines Arzneimittels zur Bereitstellung eines Gens für die Aufnahme durch eine Zelle.

17. Verwendung nach Anspruch 16, wobei das Arzneimittel zur subkutanen, intramuskulären oder intraperitonealen Verabreichung an eine Person bestimmt ist.

## Revendications

1. Préparation de gène à libération soutenue sous forme de gel comprenant un atélocollagène et un gène souhaité ou un vecteur comprenant ledit gène souhaité.

2. Préparation de gène à libération soutenue selon la revendication 1, dans laquelle ledit atélocollagène est présent en une quantité de 0,01 à 25 % p/p de la préparation.

3. Préparation de gène à libération soutenue selon la revendication 1, dans laquelle ledit atélocollagène est présent en une quantité de 0,05 à 10 % p/p de la préparation.

4. Préparation de gène à libération soutenue selon l'une quelconque des revendications 1 à 3, dans laquelle ladite préparation comprend en outre un additif.

5. Préparation de gène à libération soutenue selon la revendication 4, dans laquelle ledit atélocollagène est présent en une quantité de 2 % p/p ou plus de la préparation, et ledit additif est présent en une quantité de 5 à 900 % p/p du atélocollagène.

6. Préparation de gène à libération soutenue sous forme de film pouvant être obtenu en séchant un gel comprenant de 0,2 à 30 % p/p d'un atélocollagène, et un gène souhaité ou un vecteur comprenant ledit gène souhaité.

7. Préparation de gène à libération soutenue selon la revendication 6, dans laquelle l'atélocollagène est de 0,3 à 5 % p/p dans le gel.

8. Préparation de gène à libération soutenue selon la revendication 6, dans laquelle l'atélocollagène est de 0,4 à 2 % p/p dans le gel.

9. Préparation de gène à libération soutenue selon l'une quelconque des revendications 6 à 8, dans laquelle ledit gel comprend en outre un additif.

10. Préparation de gène à libération soutenue selon la revendication 9, dans laquelle ledit atélocollagène est de 1 % ou plus dans le gel, et l'additif est de 5 à 900 % p/p dudit atélocollagène.

11. Préparation de gène à libération soutenue sous forme de tige solide pouvant être obtenue en séchant un gel comprenant 10 à 30 % p/p d'un atélocollagène, et un gène souhaité, ou un vecteur comprenant ledit gène souhaité.

12. Préparation de gène à libération soutenue selon la revendication 11, dans laquelle ledit gel comprend en outre un additif.

13. Préparation de gène à libération soutenue selon la revendication 12, dans laquelle ledit additif est présent en une quantité de 5 à 100 % p/p de l'atélocollagène.

14. Composition pharmaceutique comprenant la préparation de gène selon l'une quelconque des revendications 1 à 13, et facultativement un véhicule pharmaceutiquement acceptable.

15. Préparation de gène à libération soutenue sous forme de tige pouvant être obtenue par le procédé comprenant les étapes consistant à :
obtenir une éponge en lyophilisant un gel comprenant de 0,05 à 5 % p/p d'atélocollagène, et un gène souhaité ou un vecteur comprenant ledit gène souhaité ;
obtenir un gel dans lequel la concentration d'atélocollagène est de 10 à 30 % p/p en ajoutant de l'eau distillée à l'éponge ;
former une tige en moulant par pression le gel ; et
sécher.

16. Utilisation de la préparation de gène selon l'une quelconque des revendications 1 à 13 ou pouvant être obtenue par le procédé de la revendication 15 pour la préparation d'une composition pharmaceutique pour délivrer un gène pour absorption par une cellule.

17. Utilisation selon la revendication 16, dans laquelle ladite composition pharmaceutique doit être administrée à un sujet par voie sous-cutanée, intramusculaire ou intrapéritonéale.
